# EUROPEAN PATENT SPECIFICATION

(11) **EP 3 647 404 B1**
(45) Date of publication and mention of the grant of the patent: **17.02.2021**
(21) Application number: 18204113.7
(22) Date of filing: 02.11.2018
(51) Int. Cl.: C12M 1/00, C12M 1/08

(54) **BIOREACTOR**
BIOREAKTOR
BIORÉACTEUR

(43) Date of publication of application: 06.05.2020
(73) Proprietor: O&N B.V., 8701 DV Bolsward (NL)
(72) Inventor: VAN VELZEN, Dick, 8711 JD WORKUM (NL); MULDER, Gerardus Rudi Maria, 8701 BL BOLSWARD (NL); OOSTERBAAN, Wiebe IJsbrand Leo, 8702 CA BOLSWARD (NL); HULSHOFF, Hendrik Jan, 8701 DV BOLSWARD (NL); STEL, Jarno, 9697 ME BLIJHAM (NL); OELEN, Jan Johannes, 7963 RP RUINEN (NL); JAGER, Filips Gustaaf Hendrik, 7861 TB OOSTERHESSELEN (NL); MAESSEN, Theodorus Antonius, 6096 CH GRATHEM (NL); VAN DRUNEN, Rudi, 2341 PH OEGSTGEEST (NL); KOEK, Charles Adrianus Gerardus, 8651 EB IJLST (NL)
(74) Representative: van Dam, Vincent

(56) References cited:
- CN-U- 202 030 744
- DE-A1- 4 416 069
- JP-A- H07 176
- US-B1- 8 569 050

## Description

The present invention relates to a bioreactor and a method for growing organisms capable of photosynthesis in an aqueous liquid. The invention also relates to a method for increasing production of algae derived lipids and/or carotenoids in said bioreactor.

Organisms capable of photosynthesis convert light energy into chemical energy by converting carbon dioxide and water into the end-products oxygen and carbohydrates. This process is energized by light.

Algae form an example of such organisms, and are well-known as efficient producers of biomass. During photosynthesis, algae utilize carbon dioxide and light in the presence of water to produce oxygen and biomass. The algae produce lipids, vegetable oils and proteins which can be used for various purposes, for example as a source for human or animal nutrition or biofuel.

Algae grow best under controlled conditions. For instance, algae are sensitive to temperature and light conditions. Algal yield may be improved by controlling the growth parameters, such as temperature, CO₂ levels, light and nutrients.

Various methods have been used to grow algae but it appears difficult to grow algae efficiently on a commercial scale.

For instance, US patent 8,569,050 discloses a bioreactor for algae comprising an outer containment vessel and a flow tube placed in the containment vessel. The outer containment vessel is provided with LEDs in acrylic spheres along the length of the interior wall of the vessel. Flow of the algae suspension is generated by a bubbier situated in the flow tube which causes the algae to travel to the top of the flow tube where the stream of algae are pushed out of the flow tube into the outer containment vessel to stream downward along the interior wall of the outer containment vessel and exterior of the flow tube where the algae are exposed to LED light from the acrylic spheres facing from the interior wall of the outer containment vessel. Once the algae suspension reaches the bottom of the bioreactor it is drawn into the flow tube with the cycle repeated.

The inventors have found that the present bioreactors, such as the one described in US patent 8,569,050, have their limitations with regard to efficiency.

### Summary of the invention

In a first aspect the invention relates to a bioreactor for growing organisms capable of photosynthesis in an aqueous liquid, comprising a tank comprising: an outer chamber having a bottom portion and a top portion and an inner chamber having a bottom portion and a top portion, wherein said inner chamber is configured within said outer chamber, wherein the bottom portion and the top portion of the inner chamber comprise one or more openings to allow fluid connection between the inner and outer chamber, and one or more spargers located in the outer chamber on the bottom portion of the outer chamber and at a lateral distance from the bottom end of the inner chamber, wherein the outer chamber comprises a plurality of LED bars distributed above said one or more spargers, each LED bar extending from the outer wall of the outer chamber in the direction of the inner chamber.

When in operation, the bioreactor is filled with an aqueous liquid with said organisms in it. In this case said tank contains an aqueous liquid with said organisms in it, wherein at the bottom and the top portion of the inner chamber one or more of said openings of the inner chamber are fully submerged in said aqueous liquid to form fluid connection between the inner and outer chamber, and wherein the plurality of LED bars in the outer chamber are submerged in said aqueous liquid.

In a second aspect the invention relates to a method for growing organisms capable of photosynthesis in an aqueous liquid in the bioreactor in operation according to the first aspect, said method comprising providing a circulation of said growing organisms between said outer and inner chamber by bubbling gas from said sparger, so that said growing organisms flow from bottom to top in the outer chamber and from top to bottom in the inner chamber, thereby exposing the organisms in the flowing aqueous liquid to said LED bars in said outer chamber.

In a third aspect the invention relates to a method for increasing production of algae derived lipids and/or carotenoids, comprising providing in the bioreactor of the first aspect a circulation of algae between said outer and inner chamber by bubbling gas from said sparger, so that said algae flow from bottom to top in the outer chamber and from top to bottom in the inner chamber, and applying stress conditions to the algae.

The use of LED bars extending from the wall of the outer chamber in the direction of the inner chamber ensures exposure of all microorganisms flowing upwards through the outer chamber, because in principle it does not matter at which position between the wall of the outer chamber and the wall of the inner chamber the microorganisms are flowing.

The use of LED bars extending from the wall of the outer chamber in the direction of the inner chamber also allows LEDs to be removably replaced from the outside of the tank without the need for disassembling the bioreactor. This allows replacement and maintenance of LEDs without the necessity of emptying the tank. This allows almost continuous operation of the bioreactor of the invention.

The LEDs in the bars have the advantage that they offer the versatility to accommodate light requirement variations amongst various microorganisms, such as in particular algal species. LEDs are also safer and more efficient than traditional lighting sources.

The use of LED light sources for algal growth however also has some disadvantages which are associated to the fact that, apart from light, LEDs produce also heat. In fact, most of the electricity in a LED is converted to heat rather than light (~ 40-50 % heat: 50-60 % light). This heat production has detrimental effects on the efficiency of the bioreactor used for growth of the algae. When LEDs are used in a bioreactor for algae, it is in general preferred that LEDs are encapsulated by a housing of light transmitting material, which housing is submerged in the algal growth medium. Because of the heat produced by the LEDs the light transmitting material of the housing is also heated. This causes caking of algae onto the surface of the housing, obscuring the LEDs from the growing algae and thus lowering the efficiency of the reactor.

The inventors have surprisingly found that the provision of one or more bubble blowing spargers, located in the outer chamber in the bottom portion of the outer chamber and at a lateral distance from the bottom end of the inner chamber, causes less caking of microorganisms on the LED bars.

At the same time the gas bubbles drive the flow cycle of the medium. This way flow of microorganisms is realized in a gentle manner which reduces stress for the microorganisms, which in its turn is beneficial for microorganism growth.

In view of the above, the provision of both the sparger (s) and the LED bars in the outer chamber results in the advantages of:
1) optimal LED light exposure of growing microorganisms;
2) easy replacement and maintenance of LEDs without the necessity of emptying the tank allowing continuous operation;
3) prevention of caking of microorganisms on the LED bars;
4) reduced stress for microorganisms.

These advantages contribute to high efficiency of the bioreactor and method of the invention.

### Short description of the figures

Fig. 1 shows a schematic representation of an embodiment of the bioreactor of the invention.
Fig. 2 shows a cross section along line B of the bioreactor of Fig. 1.
Fig. 3 shows a cross section along line A of the bioreactor of Fig. 1.

### Detailed description of the invention

In principle the bioreactor and method of the invention are suitable for all kinds of organisms which are capable of photosynthesis and growing in an aqueous medium. It is preferred that the organisms are phytoplankton or algae, such as microalgae, because these organisms produce lipids, vegetable oils and proteins which can be used for various purposes such as a source for human or animal nutrition or biofuel.

The bioreactor according to the invention comprises a tank comprising an outer chamber having a bottom and a top portion and an outer wall, and an inner chamber having a bottom portion and a top portion, wherein said inner chamber is configured within said outer chamber, wherein said one or more of said openings allow fluid connection between the inner and outer chamber via the bottom and the top portion of the inner chamber. Because the inner chamber is placed in the outer chamber, the wall of the inner chamber preferably functions as the inner wall of the outer chamber, separating the outer and inner chamber from each other.

In a preferred embodiment the inner chamber has open top and bottom ends to provide said one or more of said openings in the bottom and top portion of the inner chamber to allow fluid connection between the inner and outer chamber. When fluid connection between the inner and outer chamber is realized by an open top end and an open bottom end of the inner chamber this causes an optimal entry and exit surface in and out the inner chamber. This reduces stress for the microorganisms, which on its turn is advantageous for growth.

In a suitable embodiment the outer chamber is defined by a first vertical cylinder and the inner chamber is defined by a second vertical cylinder, wherein the second vertical cylinder is mounted into said first vertical cylinder.

In the bioreactor of the invention one or more spargers are located in the outer chamber in the bottom portion of the outer chamber and at a lateral distance from the bottom end of the inner chamber. The sparger (s) can be suitably mounted on the bottom (floor) of the outer chamber. The spargers are configured to bubble gas into the liquid growth medium containing the microorganisms in the outer chamber. Said gas may be air, nitrogen or carbon dioxide or any mixture thereof. The spargers may be in any form capable of blowing gas bubbles into the growth medium.

The bubbling gas causes a local decrease in the density of the growth medium, causing an upward flow through the outer chamber. As soon as the bubbles have reached the upper surface the gas contained in the bubbles will be released causing the density of the medium to increase. As a result the denser medium will enter the inner chamber which is not provided at its bottom with a bubble generating means and the denser medium will flow downwards through the inner chamber. The bottom of the inner chamber is in fluid contact with the medium in the outer chamber so that the down flowing medium that has reached the bottom of the inner chamber will enter the outer chamber which is provided with said bubble generating means so that the medium is forced to flow up again. This way a circular flow is created. When in operation the inner chamber is preferably fully fit into the outer chamber and fully submerged in the growth medium.

The vertical position of the sparger(s) may overlap with the vertical position of the inner chamber or be below the bottom end of the inner chamber. In accordance, the sparger(s) may be located at a position below the opening(s) in the bottom portion of the inner chamber, at the same height as the opening(s) or higher than the opening(s) as long as said sparger (s) are located below said LED bars. This is important because the inventors have found that the injection of gas below said LED bars leads to a minimum of caking of microorganisms to the LED bars.

Said one or more spargers are also located at a lateral distance from the bottom end of the inner chamber. This allows unhindered circulation of the liquid growth medium. The lateral distance is chosen such that bubbles are injected in the outer chamber and not in the inner chamber.

In one embodiment multiple spargers may be distributed in the bottom portion of the inner chamber. Preferably the spargers are distributed evenly to provide an even distribution of gas bubbles to the liquid growth medium, providing all microorganisms with an equal driving force and metabolic gas in case carbon dioxide is bubbled.

In a preferred embodiment the bioreactor comprises a sparger extending in the outer chamber in a loop at a lateral distance from the bottom end of the inner chamber and around the circumference of the inner chamber. In this embodiment a single sparger suffices. Such a loop may be easily removed and replaced for maintenance. Preferably the loop is mounted to the bottom of the outer chamber and runs around the inner chamber at a lateral distance from its bottom end at a position approximately in the centre between the wall of the inner chamber and the wall of the outer chamber. Also this provides an even distribution of gas bubbles to the liquid growth medium, providing all microorganisms with an equal driving force and metabolic gas in case carbon dioxide is bubbled.

As mentioned above, the gas bubbles cause a flow of said microorganisms upwards through the outer cylinder. During their way up, the microorganisms pass the plurality of LED bars distributed above said sparger (s), each LED bar extending from the outer wall of the outer chamber in the direction of the inner chamber. When the bioreactor is operational the bars extend in the liquid growth medium from the wall of the outer chamber to the wall of the inner chamber. The LED bars may be horizontal or angularly directed as long as preferably the major part of the distance between the wall of the outer chamber to the wall of the inner chamber is bridged by the LEDs in the bars. In order to provide optimal exposure of the microorganisms to the LED light from the bars it is preferred that the multiple LED bars extend from the outer wall of the outer chamber over essentially the full length between the outer wall of the outer chamber and the wall of the inner chamber.

As mentioned above, the use of LED lamps in the form of LED bars extending from the outer wall of the outer chamber in the direction of the inner chamber allows LEDs to be removably placed without the need for disassembling the bioreactor.

In a preferred embodiment therefore said LED bars comprise LED arrays comprising multiple LEDs in tubes of light transmitting material, which LED arrays are removable from the outside of the outer wall of the outer chamber.

In a further preferred embodiment tubes of light transmitting material extend from the outer wall of the outer chamber to the wall of the inner chamber and are attached to the wall of the inner chamber, wherein the tubes of light transmitting material each have a receiving portion at the outside of the outer wall of the outer chamber which allows inserting and removing LED arrays in any of said tubes.

It is preferred that the LED bars comprise a tubular or generally tubular housing of light transmitting material surrounding the LED arrays in spaced relationship with the LEDs on the arrays, wherein said LED arrays extend over most of the length of said tubular housing. This light transmitting material may be of any light transmitting material such as glass or plastic.

Because the bars are fully submerged in the liquid growth medium when the bioreactor is operational and are light transmitting in all directions lateral from its longitudinal axis, exposure of the microorganisms to LED light is enhanced compared to other LED systems used in the art.

For the same reason, the LED arrays are preferably configured such that LED light is emitted in all directions around the longitudinal axis of the bar.

Because the bioreactor of the invention allows control of any growth parameter, such as the light parameter, it is perfectly suitable to manipulate conditions which result in a change of metabolism of the organisms. Therefore, the bioreactor can also be used to increase production of value added products from said microorganisms. Such products comprise in particular lipids, such as poly-unsaturated fatty acids (PUFAs), such as omega 3 fatty acids. In this respect the invention also relates to a method for increasing production of algae derived lipids and/ or carotenoids, comprising providing in the bioreactor of the invention a circulation of algae between the outer and inner chamber by bubbling gas from said sparger, so that said algae flow from bottom to top in the outer chamber and from top to bottom in the inner chamber, and applying stress conditions to the algae.

The application of stress conditions may suitably involve the manipulation of growth parameters, such as temperature, light, and nutrients.

These stress conditions can lead to the accumulation of lipids in the algae. This makes it possible to produce poly-unsaturated fatty acids (PUFAs), such as omega 3 fatty acids from algae.

After stressing the algae, the algae can be harvested and processed to extract and optionally purify the lipids contained therein.

### Detailed description of the drawings

The invention will now be further elucidated in the attached drawings.

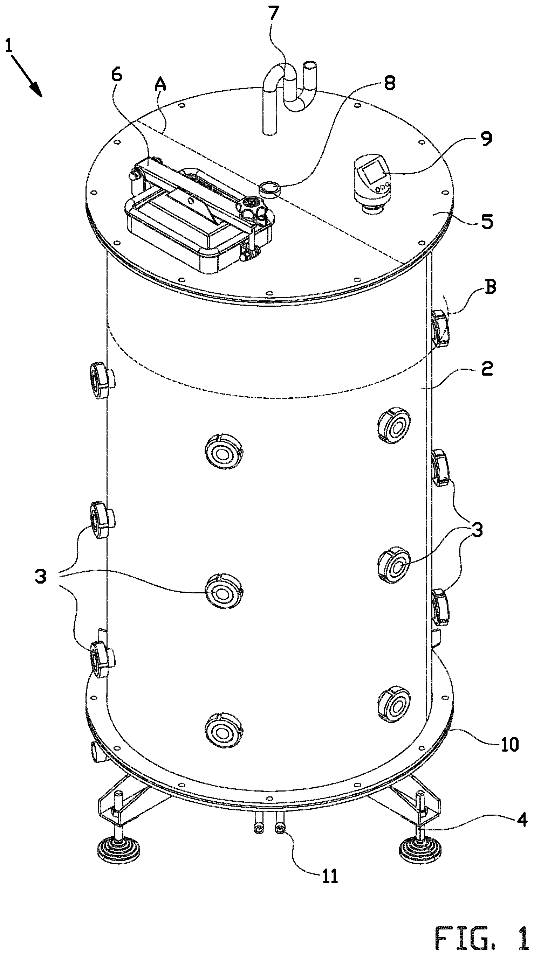

Fig. 1 shows an exemplary embodiment of a bioreactor 1 in accordance with the invention. This bioreactor comprises a cylindrical tank 2 and multiple LED bars 3 extend laterally from the outside of the tank wall into the tank. In this embodiment the tank stands on four feet 4. The tank 2 is closed with a lid 5 onto which means 6 for opening and closing the tank are provided. On the lid of this bioreactor further a pipe 7 for degassing, a sealable pipe 8 for water supply, and a water level detector 9 are provided. At the bottom of the tank a bottom plate 10 is provided. Via this bottom plate gas can be provided into the tank via pipe 11.

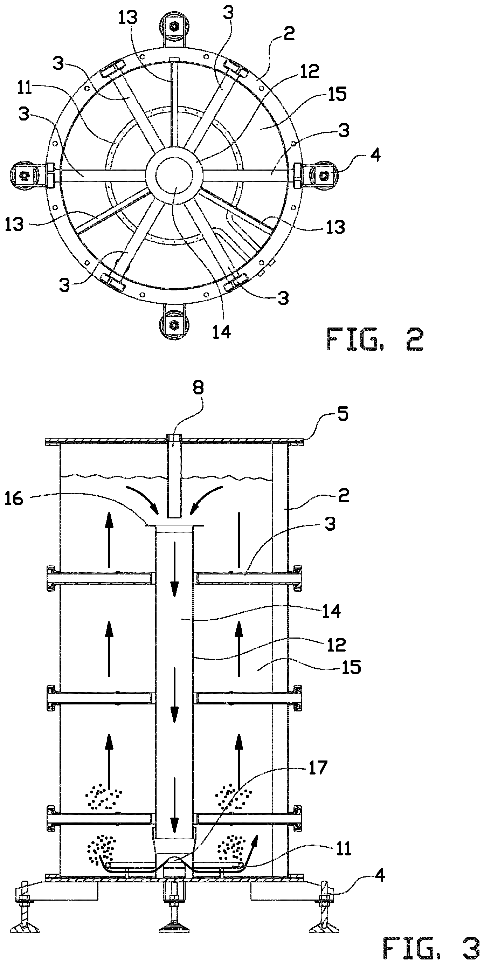

Figs. 2 and 3 show the inside of the bioreactor according to Fig. 1. Fig. 2 shows a cross section along line B of the bioreactor of Fig. 1. Fig. 3 shows a cross section along line A of the bioreactor of Fig. 1 in case the tank is filled with growth medium. The bioreactor of Figs. 1-3 allows performing a preferred embodiment of the method according to the invention wherein algae are grown by creating a circulation of algae along the LED bars submerged in the growth medium of the algae in the reactor. Inside tank 2 an inner cylinder 12 is placed supported by supporting members 13. The inner cylinder 12 defines an inner chamber 14. On the other hand the cylindrical tank 2 defines an outer chamber 15 surrounding inner chamber 14. When in operation the tank is filled with an aqueous growth medium containing algae. The level of growth medium should be such that the full inner chamber is submerged in the growth medium. To ensure that the level of growth medium is maintained at acceptable or optimal levels the tank 2 is provided with sealable pipe 8 for water supply, and a water level detector 9. Flow of the growth medium is effected by the provision of pipe 11 which extends on the bottom of the tank in a circumferential fashion as a loop around the inner cylinder at a suitable distance from it to provide a means for introducing gas such as air into the outer chamber 15. This causes a local decrease in the density of the growth medium causing an upward flow (see arrows for the direction of flow) . During the way up the algae in the growth medium are exposed to the light emitted by LED bars 3 which are provided with a tubular housing and which extend in the growth medium laterally from the wall of the tank 2 to the inner cylinder 12 and which are evenly distributed throughout the outer chamber 15. As soon as the bubbles have reached the upper surface the gas contained in the bubbles will be released on the surface of the growth medium, causing the density of the growth medium to increase locally. As a result the denser medium will enter the inner chamber 14 via opening 16. Because of its higher density the medium flows downwards. The growth medium leaves the inner chamber via bottom outlets 17 and enters the outer chamber 15. There the medium is exposed again to gas bubbles released by pipe 11, causing the medium to flow up again in the outer chamber 15, thus effecting a circulation.

## Claims

1. A bioreactor for growing organisms capable of photosynthesis in an aqueous liquid, comprising a tank comprising:
an outer chamber having a bottom portion and a top portion and an outer wall, and an inner chamber having a bottom portion and a top portion, wherein said inner chamber is configured within said outer chamber, wherein the bottom portion and the top portion of the inner chamber comprise one or more openings to allow fluid connection between the inner and outer chamber, and
one or more spargers located in the outer chamber in the bottom portion of the outer chamber and at a lateral distance from the bottom end of the inner chamber,
wherein the outer chamber comprises a plurality of LED bars distributed above said one or more spargers, each LED bar extending from the outer wall of the outer chamber in the direction of the inner chamber.

2. A bioreactor according to claim 1, wherein said inner chamber has an open top end to provide a single opening in the top portion of the inner chamber.

3. The bioreactor according to claim 1 or 2, wherein the outer chamber is defined by a first vertical cylinder and the inner chamber is defined by a second vertical cylinder, wherein the second vertical cylinder is mounted into said first vertical cylinder.

4. The bioreactor according to any of the previous claims, wherein the plurality of LED bars extend from the outer wall of the outer chamber over essentially the full length between the wall of the outer chamber and the wall of the inner chamber.

5. The bioreactor according to any of the previous claims, comprising a sparger extending in the outer chamber in a loop at a lateral distance from the bottom end of the inner chamber and around the circumference of the inner chamber.

6. The bioreactor according to any of the previous claims, wherein said LED bars comprise LEDs in tubes of light transmitting material, which LEDs are removable from the outside of the outer wall of the outer chamber.

7. The bioreactor according to claim 6, wherein the tubes of light transmitting material extend from the outer wall of the outer chamber to the wall of the inner chamber and are attached to the wall of the inner chamber, wherein the tubes of light transmitting material each have a receiving portion at the outside of the outer wall of the outer chamber which allows inserting and removing LEDs in any of said tubes.

8. Bioreactor according to any of the previous claims, wherein the organisms capable of photosynthesis are phytoplankton.

9. Bioreactor according to any of the claims 1 to 7, wherein the organisms capable of photosynthesis are algae.

10. The bioreactor according to any of the previous claims, wherein said tank contains an aqueous liquid with said organisms in it, wherein at the bottom portion and the top portion of the inner chamber one or more of said openings in the inner chamber are fully submerged in said aqueous liquid to form fluid connection between the inner and outer chamber, and wherein the plurality of LED bars are submerged in said aqueous liquid.

11. A method for growing organisms capable of photosynthesis in an aqueous liquid in the bioreactor according to any of the previous claims, said method comprising providing a circulation of said growing organisms between said outer and inner chamber by bubbling gas from said sparger, so that said growing organisms flow from bottom to top in the outer chamber and from top to bottom in the inner chamber, thereby exposing the organisms in the flowing aqueous liquid to said LED bars in said outer chamber.

12. The method according to claim 11 wherein said gas is air, nitrogen or carbon dioxide or any mixture thereof.

13. A method for increasing production of algae derived lipids and/or carotenoids, comprising providing in the bioreactor of any of the claims 1 to 10 a circulation of algae between said outer and inner chamber by bubbling gas from said sparger, so that said algae flow from bottom to top in the outer chamber and from top to bottom in the inner chamber; and
applying stress conditions to the algae.

14. Method according to claim 13, wherein the application of stress conditions involves the manipulation of growth parameters, such as temperature, light, and nutrients.

15. Method according to claim 13 or 14, wherein the lipids comprise poly-unsaturated fatty acids (PUFAs), such as omega 3 fatty acids.

## Patentansprüche

1. Ein Bioreaktor zum Wachsen-Lassen von Organismen, die zu Photosynthese in der Lage sind, in einer wässerigen Flüssigkeit, der einen Tank aufweist, mit folgenden Merkmalen:
einer äußeren Kammer mit einem unteren Abschnitt und einem oberen Abschnitt und einer Außenwand, sowie einer inneren Kammer mit einem unteren Abschnitt und einem oberen Abschnitt, wobei die innere Kammer innerhalb der äußeren Kammer ausgebildet ist, wobei der untere Abschnitt und der obere Abschnitt der inneren Kammer eine oder mehr Öffnungen aufweisen, um eine Fluidverbindung zwischen der inneren und der äußeren Kammer zu ermöglichen, und
einem oder mehr Gasverteilern, die sich in der äußeren Kammer in dem unteren Abschnitt der äußeren Kammer und in einer lateralen Entfernung von dem unteren Ende der inneren Kammer befinden,
wobei die äußere Kammer eine Mehrzahl von LED-Leisten aufweist, die oberhalb des einen oder der mehr Gasverteiler verteilt sind, wobei sich jede LED-Leiste von der Außenwand der äußeren Kammer in Richtung der inneren Kammer erstreckt.

2. Ein Bioreaktor gemäß Anspruch 1, bei dem die innere Kammer ein offenes oberes Ende aufweist, um eine einzelne Öffnung in dem oberen Abschnitt der inneren Kammer bereitzustellen.

3. Der Bioreaktor gemäß Anspruch 1 oder 2, bei dem die äußere Kammer definiert ist durch einen ersten vertikalen Zylinder und die innere Kammer definiert ist durch einen zweiten vertikalen Zylinder, wobei der zweite vertikale Zylinder in dem ersten vertikalen Zylinder befestigt ist.

4. Der Bioreaktor gemäß einem der vorherigen Ansprüche, bei dem sich die Mehrzahl von LED-Leisten von der Außenwand der äußeren Kammer über im Wesentlichen die volle Länge zwischen der Wand der äußeren Kammer und der Wand der inneren Kammer erstreckt.

5. Der Bioreaktor gemäß einem der vorherigen Ansprüche, der einen Gasverteiler aufweist, der sich in der äußeren Kammer in einer Schleife in einer lateralen Entfernung von dem unteren Ende der inneren Kammer und um den Umfang der inneren Kammer herum erstreckt.

6. Der Bioreaktor gemäß einem der vorherigen Ansprüche, bei dem die LED-Leisten LEDs in Röhren aus einem lichtdurchlässigen Material aufweisen, wobei diese LEDs von der Außenseite der Außenwand der äußeren Kammer entfernbar sind.

7. Der Bioreaktor gemäß Anspruch 6, bei dem sich die Röhren aus lichtdurchlässigem Material von der Außenwand der äußeren Kammer zu der Wand der inneren Kammer erstrecken und an der Wand der inneren Kammer angebracht sind, wobei die Röhren aus lichtdurchlässigem Material jeweils einen Aufnahmeabschnitt an der Außenseite der Außenwand der äußeren Kammer aufweisen, der ein Einführen und Entfernen von LEDs in jede der Röhren ermöglicht.

8. Bioreaktor gemäß einem der vorherigen Ansprüche, bei dem die Organismen, die zu Photosynthese in der Lage sind, Phytoplankton sind.

9. Bioreaktor gemäß einem der Ansprüche 1 bis 7, bei dem die Organismen, die zu Photosynthese in der Lage sind, Algen sind.

10. Der Bioreaktor gemäß einem der vorherigen Ansprüche, bei dem der Tank eine wässerige Flüssigkeit mit den Organismen in derselben enthält, wobei an dem unteren Abschnitt und dem oberen Abschnitt der inneren Kammer eine oder mehr der Öffnungen in der inneren Kammer vollständig in die wässerige Flüssigkeit eingetaucht sind, um eine Fluidverbindung zwischen der inneren und der äußeren Kammer zu bilden, und wobei die Mehrzahl von LED-Leisten in die wässerige Flüssigkeit eingetaucht ist.

11. Ein Verfahren zum Wachsen-Lassen von Organismen, die zu Photosynthese in der Lage sind, in einer wässerigen Flüssigkeit in dem Bioreaktor gemäß einem der vorherigen Ansprüche, wobei das Verfahren ein Bereitstellen einer Zirkulation der wachsenden Organismen zwischen der äußeren und der inneren Kammer durch Sprudeln-Lassen von Gas aus dem Gasverteiler aufweist, so dass die wachsenden Organismen in der äußeren Kammer von unten nach oben und in der inneren Kammer von oben nach unten strömen, wodurch die Organismen in der strömenden wässerigen Flüssigkeit den LED-Leisten in der äußeren Kammer ausgesetzt werden.

12. Das Verfahren gemäß Anspruch 11, bei dem das Gas Luft, Stickstoff oder Kohlendioxid oder eine Mischung derselben ist.

13. Ein Verfahren zum Erhöhen der Produktion von von Algen hergeleiteten Lipiden und/oder Carotenoiden, das ein Bereitstellen, in dem Bioreaktor gemäß einem der Ansprüche 1 bis 10, einer Zirkulation von Algen zwischen der äußeren und der inneren Kammer durch Sprudeln-Lassen von Gas aus dem Gasverteiler aufweist, so dass die Algen in der äußeren Kammer von unten nach oben und in der inneren Kammer von oben nach unten strömen; und
Anlegen von Belastungsbedingungen für die Algen.

14. Verfahren gemäß Anspruch 13, bei dem das Anlegen von Belastungsbedingungen die Manipulierung von Wachstumsparametern, wie z. B. Temperatur, Licht und Nährstoffen, beinhaltet.

15. Verfahren gemäß Anspruch 13 oder 14, bei dem die Lipide mehrfach ungesättigte Fettsäuren (PUFAs), wie z. B. Omega-3-Fettsäuren, aufweisen.

## Revendications

1. Bioréacteur pour faire croître des organismes disposant d'une capacité de photosynthèse dans un liquide aqueux, comprenant un réservoir qui comprend :
une chambre externe qui comporte une partie de fond et une partie de sommet ainsi qu'une paroi externe, et une chambre interne qui comporte une partie de fond et une partie de sommet, dans lequel ladite chambre interne est configurée à l'intérieur de ladite chambre externe, dans lequel la partie de fond et la partie de sommet de la chambre interne comprennent une ou plusieurs ouverture(s) pour permettre une connexion en termes de fluide entre la chambre interne et la chambre externe ; et
un ou plusieurs aérateur(s) qui est/sont localisé(s) à l'intérieur de la chambre externe dans la partie de fond de la chambre externe et à une distance latérale de l'extrémité de fond de la chambre interne ;
dans lequel la chambre externe comprend une pluralité de barres de LED qui sont distribuées au-dessus desdits un ou plusieurs aérateurs, chaque barre de LED s'étendant depuis la paroi externe de la chambre externe dans la direction de la chambre interne.

2. Bioréacteur selon la revendication 1, dans lequel ladite chambre interne comporte une extrémité de sommet ouverte pour constituer une unique ouverture dans la partie de sommet de la chambre interne.

3. Bioréacteur selon la revendication 1 ou 2, dans lequel la chambre externe est définie par un premier cylindre vertical et la chambre interne est définie par un second cylindre vertical, dans lequel le second cylindre vertical est monté à l'intérieur dudit premier cylindre vertical.

4. Bioréacteur selon l'une quelconque des revendications précédentes, dans lequel les barres de la pluralité de barres de LED s'étendent depuis la paroi externe de la chambre externe au-dessus essentiellement de la totalité de la longueur entre la paroi de la chambre externe et la paroi de la chambre interne.

5. Bioréacteur selon l'une quelconque des revendications précédentes, comprenant un aérateur qui s'étend à l'intérieur de la chambre externe selon une boucle à une distance latérale de l'extrémité de fond de la chambre interne et autour de la circonférence de la chambre interne.

6. Bioréacteur selon l'une quelconque des revendications précédentes, dans lequel lesdites barres de LED comprennent des LED à l'intérieur de tubes en un matériau laissant passer la lumière, lesquelles LED peuvent être enlevées de l'extérieur de la paroi externe de la chambre externe.

7. Bioréacteur selon la revendication 6, dans lequel les tubes en un matériau laissant passer la lumière s'étendent depuis la paroi externe de la chambre externe jusqu'à la paroi de la chambre interne et sont fixés à la paroi de la chambre interne, dans lequel les tubes en un matériau laissant passer la lumière comportent chacun une partie de réception au niveau de l'extérieur de la paroi externe de la chambre externe, laquelle partie de réception permet l'insertion et l'enlèvement des LED dans n'importe lequel desdits tubes.

8. Bioréacteur selon l'une quelconque des revendications précédentes, dans lequel les organismes disposant d'une capacité de photosynthèse sont du phytoplancton.

9. Bioréacteur selon l'une quelconque des revendications 1 à 7, dans lequel les organismes disposant d'une capacité de photosynthèse sont des algues.

10. Bioréacteur selon l'une quelconque des revendications précédentes, dans lequel ledit réservoir contient un liquide aqueux dans lequel lesdits organismes sont placés, dans lequel, au niveau de la partie de fond et de la partie de sommet de la chambre interne, une ou plusieurs desdites ouvertures qui sont ménagées dans la chambre interne est/sont complètement submergée(s) dans ledit liquide aqueux de manière à former une connexion en termes de fluide entre la chambre interne et la chambre externe, et dans lequel les barres de la pluralité de barres de LED sont submergées dans ledit liquide aqueux.

11. Procédé pour faire croître des organismes disposant d'une capacité de photosynthèse dans un liquide aqueux dans le bioréacteur selon l'une quelconque des revendications précédentes, ledit procédé comprenant la réalisation d'une circulation desdits organismes en cours de croissance entre ladite chambre externe et ladite chambre interne en insufflant des bulles de gaz en provenance dudit aérateur, de telle sorte que lesdits organismes en cours de croissance circulent depuis le fond jusqu'au sommet à l'intérieur de la chambre externe et depuis le sommet jusqu'au fond à l'intérieur de la chambre interne, d'où ainsi l'exposition des organismes dans le liquide aqueux en cours d'écoulement auxdites barres de LED à l'intérieur de ladite chambre externe.

12. Procédé selon la revendication 11, dans lequel ledit gaz est de l'air, de l'azote ou du dioxyde de carbone ou un quelconque mélange de ceux-ci.

13. Procédé pour augmenter la production de lipides et/ou de caroténoïdes dérivés à partir d'algues, comprenant la réalisation, à l'intérieur du bioréacteur selon l'une quelconque des revendications 1 à 10, d'une circulation d'algues entre ladite chambre externe et ladite chambre interne en insufflant des bulles de gaz en provenance dudit aérateur, de telle sorte que lesdites algues circulent depuis le fond jusqu'au sommet à l'intérieur de la chambre externe et depuis le sommet jusqu'au fond à l'intérieur de la chambre interne ; et
l'application de conditions de contrainte aux algues.

14. Procédé selon la revendication 13, dans lequel l'application de conditions de contrainte met en jeu la manipulation de paramètres de croissance tels que la température, la lumière et les nutriments.

15. Procédé selon la revendication 13 ou 14, dans lequel les lipides comprennent des acides gras polyinsaturés (PUFA), tels que des acides gras oméga 3.
